(19) Europäisches Patentamt
European Patent Office
Office européen des brevets

(11) Numéro de publication : **0 565 463 A1**

(12) **DEMANDE DE BREVET EUROPEEN**

(21) Numéro de dépôt : **93420148.4**

(22) Date de dépôt : **07.04.93**

(51) Int. Cl.[5] : **C07D 521/00,** C07D 405/08, C07D 405/06, C07C 49/237, C07D 303/08, C07D 303/12, C07D 407/04

(30) Priorité : **08.04.92 FR 9204524**

(43) Date de publication de la demande : **13.10.93 Bulletin 93/41**

(84) Etats contractants désignés : **AT BE CH DE DK ES FR GB GR IE IT LI LU NL PT SE**

(71) Demandeur : **RHONE-POULENC AGROCHIMIE
14-20, rue Pierre Baizet
F-69009 Lyon (FR)**

(72) Inventeur : **Hutt, Jean
200 bis, rue de St Cyr
F-69009 Lyon (FR)**
Inventeur : **Latorse, Marie-Pascale
2 ter, rue Louis Bouquet
F-69009 Lyon (FR)**
Inventeur : **Veryat, Christine
21, rue Nervieux
F-69450 St Cyr au Mont d'Or (FR)**

(74) Mandataire : **Chrétien, François et al
RHONE-POULENC AGROCHIMIE- DPI B.P. 9163
F-69263 Lyon Cédex 09 (FR)**

(54) **Dérivés triazole et imidazole fongicides.**

(57) L'invention concerne de nouveaux composés à groupement triazole ou imidazole à usage phytosanitaire de formule générale :

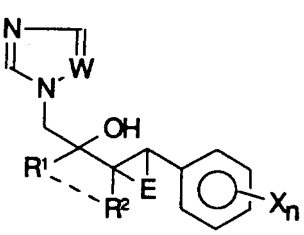

dans laquelle :

W est N ou CH ;
E est $CH_2$ ou O ;
X est un halogène ou un radical alkyle ;
n est un entier de 0 à 5 ;
R1 est un alkyle, un cycloalkyle ou un aryle ;
R2 est un alkyle ou un perfluoroalkyle.

L'invention concerne également la préparation de ces composés et leur utilisation comme fongicides à spectre large.

EP 0 565 463 A1

La présente invention concerne de nouveaux composés à groupement triazole ou imidazole à usage phytosanitaire.Elle concerne également les procédés de préparation de ces composés et les composés éventuellement utilisables comme intermédiaires dans les procédés de préparation.Elle concerne enfin l'utilisation fongicide de ces composés, les compositions fongicides à base de ces composés et les procédés, pour lutter contre les maladies fongiques des cultures, utilisant ces composés.Elle concerne également un produit de multiplication des plantes cultivées qui a subi un traitement de protection par un produit de l'invention.

Un des buts de la présente invention est de proposer des composés présentant une activité améliorée dans le traitement des maladies fongiques.

Un autre but est de proposer des composés présentant un spectre d'utilisation également amélioré dans le domaine des maladies fongiques.

De nombreux produits à groupes triazole, notamment des fongicides, sont déjà connus.En particulier, par les demandes de brevets EP 151084, EP 246982, EP 121979, EP 89100 on connait des fongicides triazoles à cycle tétrahydrofuranne. Par les demandes de brevets EP 272895, EP 267778, EP 378953, DE 3630840, BE 867245 on connait des fongicides triazoles à cycle cyclopentane.Par la demande de brevet EP 324646 et le brevet US 4684396, on connait des fongicides triazoles à groupe cycloalcane.

D'autres composés triazole ou imidazole fongicides sont décrits dans la littérature brevets.Certains triazoles ou imidazoles à groupe cyclopropyl sont connus par les demandes de brevets BE 886128, EP 40345, EP 47594, EP 52424, EP 143384, EP 180136, EP 180838, EP 212605 et DE 3909862.

Certains triazoles ou imidazoles à groupe oxirane sont décrits dans les demandes de brevets EP 251086 et DE 3819053.

Ainsi compte tenu de cet état de la technique, un but de la présente invention est de proposer d'autres composés triazoles ou imidazoles fongicides à large spectre utiles notamment dans le traitement des maladies du pied comme le piétin verse ou de la feuille comme l'oïdium, la septoriose, la piriculariose, les fusarioses, la rhynchosporiose, le rhizoctone, des maladies provoquées par les champignons pathogènes tels que Botrytis, Phoma, Aschochyta, dans les cultures aussi diverses que les céréales, la vigne, le riz, le mais et le soja par exemple.

Il a été maintenant trouvé que les buts précités pouvaient être atteints grâce aux composés selon l'invention, qui sont de nouveaux dérivés à groupe triazole ou imidazole et à groupe oxirane ou cyclopropyl, éventuellement substitué. Ces composés ont pour formule générale VII :

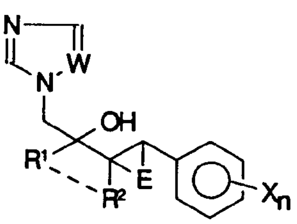

dans laquelle :

- $R^1$ représente un radical alkyle de 1 à 4 atomes de carbone, linéaire ou ramifié; cycloalkyle de 3 à 6 atomes de carbone; alkylidène, hydroxyalkyle, carboxyalkyle de 1 à 4 atomes de carbone, ces radicaux étant éventuellement substitués par un ou plusieurs atomes d'halogène; aryle, choisi dans le groupe comprenant phényle, naphtyle, éventuellement substitué par un ou plusieurs groupes choisis parmi halogène, nitro, cyano, amino, alkyle ou alkoxy de 1 à 4 atomes de carbone éventuellement substitués par un ou plusieurs atomes d'halogène, alkylamino, la partie alkyle ayant de 1 à 4 atomes de carbone éventuellement substitués par un ou plusieurs atomes d'halogène; aralkyle, dont la partie alkyle possède de 1 à 4 atomes de carbone, linéaire ou ramifié et dont la partie aryle est définie comme ci-dessus;
- $R^2$ représente un radical alkyle ou perhalogénoalkyle de 1 à 4 atomes de carbone, linéaire ou ramifié, ou un atome d'halogène, de préférence chlore ou fluor, lorsque E est autre qu'un atome d'oxygène;
- $R^1$ et $R^2$, ensemble, peuvent former un radical $-(CH_2)_3-$, $-(CH_2)_4-$, $-(CH_2)_5-$, conduisant ainsi à un cycle à 5, 6 ou 7 atomes de carbone, cycle dont chaque hydrogène peut éventuellement être substitué par un radical choisi dans le groupe comprenant les atomes d'halogène, les radicaux alkyle, de 1 à 4 atomes de carbone, linéaire ou ramifié, éventuellement substitués par un ou plusieurs atomes d'halogène;
- W est un radical CH ou l'atome d'azote;
- E représente un atome d'oxygène ou un radical $CH_2$;
- X représente un atome d'halogène F, Cl, Br; un radical alkyle, de 1 à 4 atomes de carbone, linéaire ou

ramifié, éventuellement substitués par un ou plusieurs atomes d'halogène; alkylamino, la partie alkyl ayant de 1 à 4 atomes de carbone éventuellement substitués par un ou plusieurs atomes d'halogène; alcoxy de 1 à 4 atomes de carbone éventuellement substitués par un ou plusieurs atomes d'halogène; phénoxy ou benzyloxy éventuellement substitué par un ou plusieurs halogènes; nitro; cyano;

- n est un entier égal à 1, 2, 3, 4 ou 5, avec, quand cet entier est supérieur ou égal à 2, la possibilité que les groupements X ne soient pas identiques.

L'invention concerne également les formes salifiées des composés ci-dessus définis.Les formes salifiées sont les formes acceptables en agriculture parmi lesquelles on peut citer les chlorhydrates, les sulfates, les oxalates, les nitrates ou les alkyl- ou aryl-sulfonates ainsi que les complexes d'addition de ces composés avec des sels métalliques, et notamment des sels de fer, chrome, cuivre, manganèse, zinc, cobalt, étain, magnésium et aluminium.

Les composés de l'invention et les composés éventuellement utilisables à titre d'intermédiaires dans les procédés de préparation, et qui seront définis à l'occasion de la description de ces procédés, peuvent exister sous une ou plusieurs formes d'isomères selon le nombre de centres asymétriques de la molécule. L'invention concerne donc aussi bien tous les isomères optiques que leur mélanges racémiques et les diastéréoisomères correspondants, séparés ou en mélange. La séparation des isomères optiques et/ou des diastéréoisomères peut s'effectuer selon des méthodes connues en soi.

Parmi les composés de l'invention, on préfèrera en vue des applications fongicides ceux pour lesquels W est l'atome d'azote, donc les composés triazoles.

De façon encore plus avantageuse, on choisira des composés triazoles ayant un groupe oxirane ou cyclopropane.

Finalement, les composés avec X qui représente un halogène, de préférence le chlore, R1 qui est un groupe propyle ou butyle, linéaire ou ramifié, et R2 qui est un radical méthyl ou éthyl, ou avec R1et R2 formant ensemble un cycle à 5 ou 6 atomes de carbone, constituent un groupe de composés préférés parmi les composés de l'invention à groupements triazoles et oxirane ou cyclopropane.

L'invention concerne également l'utilisation préventive ou curative des composés ci-dessus définis comme fongicides ainsi que les compositions fongicides.

Les composés de l'invention peuvent être utilisés en traitement foliaire, en traitement de sol ou, vu l'absence de phytotoxicité, en traitement de semences.

L'invention concerne également un procédé de traitement des cultures atteintes ou susceptibles d'être atteintes par les maladies fongiques caractérisé en ce l'on applique sur les feuilles une dose efficace d'un composé de l'invention.

Les composés s'appliquent avantageusement à des doses de 0,002 à 5 kg/ha, et plus spécifiquement de 0,005 à 1 kg/ha.

L'invention concerne en outre un produit de multiplication des plantes cultivées qui a subi un traitement de protection avec un composé de l'invention.

On désigne par produit de multiplication toutes les partie génératives de la plante que l'on peut utiliser pour la multiplication de celle-ci.On citera par exemple les graines (semence au sens étroit), les racines, les fruits, les tubercules, les bulbes, les rhizomes, les partie de tige, les plants, pousses et autres partie de plantes.On mentionnera également les plantes germées et les jeunes plants qui doivent être transplantés apis germination ou après sorbe de terre.Ces jeunes plants peuvent être protégés avant la transplantation par un traitement total ou partiel par immersion.

En général, les composés seront appliqués à raison de 0,1 à 500 g par quintal de graines.Les graines subiront alors un traitement avec les composés selon l'invention par pelliculage ou enrobage.Les brevets US 3989501 et FR 2588442 décrivent de telles formes d'application.

La présente invention a également pour objet des procédés de préparation des composés selon l'invention ainsi que les composés éventuellement utilisables à titre d'intermédiaires dans les procédés de préparation.

Un premier ensemble de procédés, dont on donne le schéma A ci-après, fait intervenir les composés intermédiaires de formules II, III, IV, V, VI, VIII, IX, dans lesquelles les substituants W, E, R1, R2, X et n ont la même signification que dans la formule générale VII ci-dessus.

SCHEMA REACTIONNEL A:

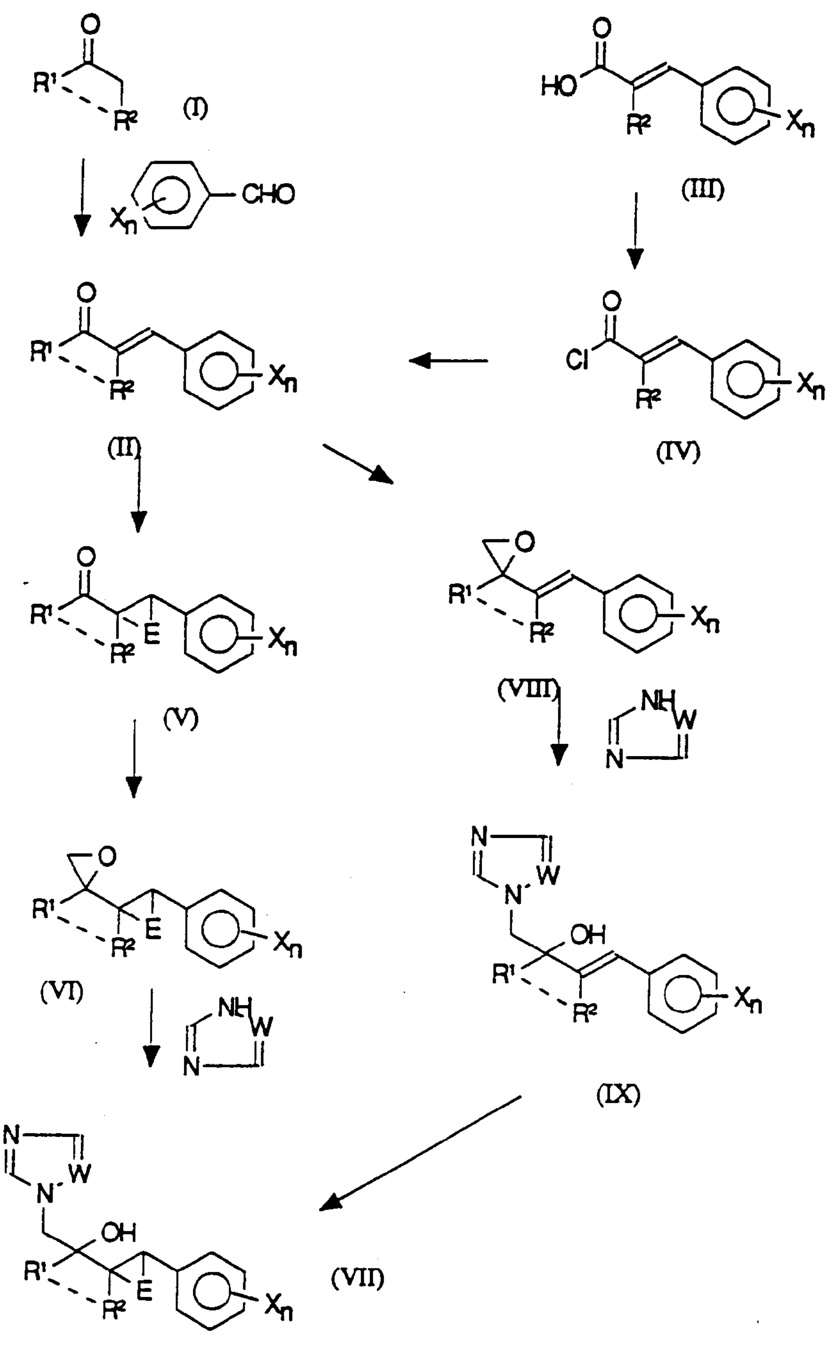

(I) (III) (II) (IV) (V) (VIII) (VI) (IX) (VII)

Le procédé consiste à condenser une cétone de formule générale (I)

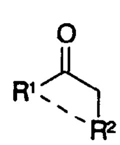

(I)

avec un aldéhyde de formule :

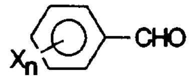

en présence d'une base telle que les hydroxydes de métaux alcalins ou alcalino-terreux, alcoolates de métaux alcalins, hydrure de sodium, amidures de lithium, ou d'un acide tel que l'acide sulfique, acétique, chlorhydrique, bromhydrique, p-toluènesulfonique, méthanesulfonique dans un solvant comme un alcool $C_1$ à $C_5$ ou un éther ou un hydrocarbure aromatique, au moyen de la réaction bien connue d'aldolisation-crotonisation de façon à obtenir le composé de formule (II)

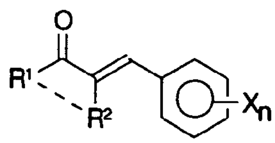

(II)

de façon générale, les cétones de formule (II), dans lesquellesR1 et R2 forment ensemble un cycle, peuvent être obtenues selon les méthodes décrites dans la demande EP 378 953. Elles peuvent être en particulier obtenues par réaction d'un aldéhyde de formule:

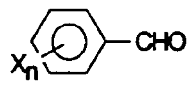

avec un anhydride de formule $(R^2CH_2CO)_2O$ et le sel de sodium ou potassium de l'acide carboxylique correspondant $R^2CH_2CO_2H$ selon la réaction de Perkin décrite dans Org. React. 1 , 251 (1942) pour obtenir un dérivé de l'acide cinnamique de formule (III)

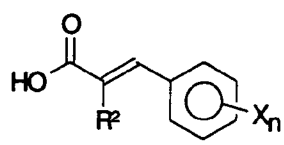

(III)

Le composé (III) est transformé en chlorure d'acide (IV)

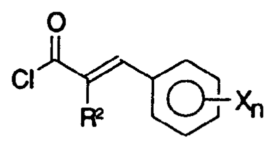

(IV)

à l'aide de chlorure de thionyle, trichlorure de phosphore, pentachlorure de phosphore, chlorure d'oxalyle ou du complexe triphénylphosphine - tétrachlorure de carbone.

La cétone (II) est ensuite obtenue par :

- couplage du chlorure d'acide (IV) avec un organomagnésien $R^1MgCl$ ou un organozincique $R^1{}_2Zn$ dans un solvant tel que l'éther ou le tétrahydrofurane avec un intervalle de température de -78° à +25°C en l'absence ou en présence de cuivre et de chlorure cuivreux selon la méthode décrite dans la littérature (J. E. Dubois, M. Boussu, Tetrahedron Lett. 1970, 2523).
- couplage du chlorure d'acide (IV) avec l'énolate de lithium, de sodium ou de potassium de la butyrolac-

tone dans le THF, suivi d'une extrusion de dioxyde de carbone en présence de LiCl, LiBr, NaCl ou NaBr dans un solvant tel que le dimethylsulfoxyde, N,N-dimethylformamide ou N-méthyl-2-pyrrolidinone à une température comprise entre l'ambiante et le reflux du solvant selon la méthode décrite dans la littérature (S. Takei, Y. Kawano, Tetrahedron Lett. 1975, 4389).

- couplage du chlorure d'acide (IV) avec le dianion lithié de l'acide carboxylique $R^1COOH$ (à condition que $R^1$ possède au moins un atome d'hydrogène) dans un solvant tel que le tetrahydrofurane ou l'éther suivi d'un traitement acide décarboxylant. selon la méthode décrite dans la littérature (P. E. Pfeffer, L. S. Silbert, J. M. Chirinko, J. Org. Chem. 1972, 37, 451)
- couplage de l'amide de manière en soi connue à partir du chlorure d'acide par réaction avec une amine, de préférence secondaire, avec un organomagnésien ou organolithien, come décrit par S.M.Weinreb et al, Tetrahedron Letters, 1981, vol 22, pp 3815-3818.

Le composé de formule (II) est mis à réagir avec un ylure de sulfoxonium, de formule $(CH_3)_2S(O)=CH_2$, obtenu par action d'une base telle qu'un alcoolate de métal alcalin, NaH, ou les hydroxydes de métaux alcalins sur un halogénure, par exemple de l'iodure de trimethylsulfoxonium $((CH_3)_3SO^+, I^-)$ dans un solvant tel que le dimethylsulfoxyde, la N,N-dimethylformamide, la N-methyl-2-pyrrolidinone, l'acétonitrile, ou un mélange de ces solvants avec de l'alcool tert-butylique ou de l'alcool tert-amylique dans un rapport 50:50 à 90:10. à une température comprise entre 20° et 100°C pour obtenir le composé de formule (V). Le composé de formule (II) peut être également mis à réagir avec du peroxyde d'hydrogène ou de l'hydroperoxyde de tert-butyle en présence d'une base telle qu'un hydroxyde de métal alcalin dans un mélange eau-alcool $C_1$-$C_5$ dans un intervalle de température compris entre 0°C et le reflux du solvant pour obtenir le composé de formule (V)

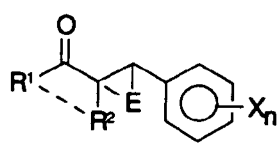

(V)

Ledit composé (V) est mis à réagir avec un ylure de sulfonium, de formule $(CH_3)_2S=CH_2$, obtenu par action d'une base telle que les hydroxydes de métaux alcalins, NaH, les alcoolates de métaux alkalins sur $((CH_3)_3S^+,X^-)$ (X = Cl, Br, I, $CH_3SO_4$) dans un solvant tel que le dimethylsulfoxyde, la N,N-dimethylformamide, la N-methyl-2-pyrrolidinone, l'acétonitrile ou un mélange dichlorométhane-eau ou toluène-eau à une température comprise entre -10°c et +50°C pour obtenir un oxirane de formule (VI)

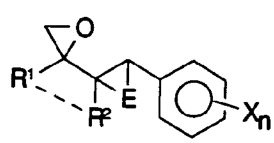

(VI)

Cet oxirane peut également être obtenu en faisant réagir un halométhyllithien sur le composé de formule (V) comme décrit dans Bull. Soc. Chim. de France 1986, vol 3, pp 470-477.

L'oxirane (VI) est mis à réagir avec un hétérocycle azoté de formule générale

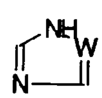

en présence de bases telles que NaH, les hydroxydes de métaux alcalins, les alcoolates de métaux alcalins, les carbonates de sodium ou de potassium dans un solvant comme la N-méthyl-2-pyrrolidinone, la N,N-dimethylformamide, le diméthylsulfoxyde, les alcools en $C_1$-$C_5$, les nitriles dans un intervalle de température

compris entre +25°C et le reflux du solvant, pour obtenir un composé de formule (VII):

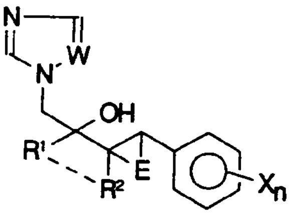

(VII)

Les composés de formule (VII) peuvent également être obtenus à partir du composé (II) comme décrit dans le brevet EP-378953-A par réaction avec un ylure de sulfonium, de formule $(CH_3)_2S{=}CH_2$, obtenu par action d'une base telle que les hydroxydes de métaux alcalins, NaH, les alcoolates de métaux alcalins sur $((CH_3)_3S^+,X^-)$ (X = Cl, Br, I, $CH_3SO_4$) dans un solvant tel que le dimethylsulfoxyde, la N,N-dimethylformamide, la N-methyl-2-pyrrolidinone, l'acétonitrile ou un mélange dichlorométhane-eau ou toluène-eau à une température comprise entre -10°c et +50°C pour obtenir un oxirane de formule (VIII)

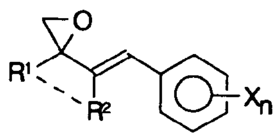

(VIII)

Ledit oxirane est mis à réagir avec un hétérocycle azoté de formule générale

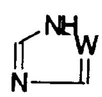

en présence de bases telles que NaH, les hydroxydes de métaux alcalins, les alcoolates de métaux alcalins, les carbonates de sodium ou de potassium dans un solvant comme la N-méthyl-2-pyrrolidinone, la N,N-dimethylformamide, le diméthylsulfoxyde, les alcools en $C_1$-$C_5$, les nitriles dans un intervalle de température compris entre +25°C et le reflux du solvant, pour obtenir un composé de formule (IX)

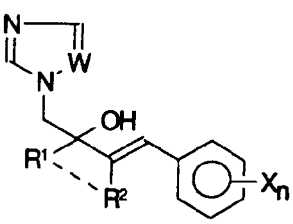

(IX)

Dans le cas où E est CH2, le composé de formule (IX) est soumis à une réaction de cyclopropanation du type Simmons Smith (cf Organic Reactions, 1973, vol 20, p 1-131) avec un dihalogénométhane, de préférence diiodo ou dibromo. Une variante consiste à réaliser la réaction sous ultra-sons comme décrit dans Tetrahedron Letters, 1982, vol 23, pp 2729-2732 ou selon la demande britannique 2 191 774.

Dans le cas où E est un atome d'oxygène, le composé de formule (IX) est soumis à une oxydation avec de l'acide méta-chloro perbenzoïque ou de l'hydroperoxyde de tert-butyle dans un solvant tel que les solvants halogénés, les hydrocarbures pour obtenir le composé selon l'invention de formule (VII).

Un second ensemble de procédés, dont on donne le schéma B ci-après, fait intervenir les composés intermédiaires de formules X, XI, XII et XIII, dans lesquelles les substituants W, E, R1, R2, X et n ont la même

signification que dans la formule générale VII ci-dessus et:

- R3 représente un atome d'halogène, de préférence de chlore ou de brome, ou un groupe Y-R4, dans lequel Y représente un atome de d'oxygène, de soufre ou d'azote et R4 représente un atome d'hydrogène, un radical alkyle linéaire ou ramifié de 1 à 4 atomes de carbone, cycloalkyle de 3 à 6 atomes de carbone, phényle ou napthyle, éventuellement substitué par 1 à 5 substituants choisi dans le groupe comprenant un atome d'halogène, amino, alkyle ou alkoxy de 1 à 4 atomes de carbone, ou encore phénylalkyle ou naphthylalkyle, dont la partie alkyle comprend de 1 à 4 atomes de carbone. Dans le cas où Y est un atome d'azote, il porte en plus de R4 un second substituant, identique ou différent de R4 et pouvant avoir les mêmes significations.
- R'3 représente un atome d'halogène(identique à R3, quand R3 est un atome d'halogène), ou
- un sel de sulfonium ou de sulfoxonium, de préférence C1-C4 alkylsulfonium ou C1-C4 alkyl sulfoxonium, lorsque Y est un atome de soufre,
- un sel d'ammonium(de préférence C1-C4 alkylammonium ), lorsque Y est un atome d'azote ou
- un groupe alkyl(de préférence méthyl)sulfonate, phényl(de préférence tolyl)sulfonate ou naphthylsulfonate, halogénoalkylcarbonyloxy(de préférence trifluoromethylcarbonyloxy), lorsque Y est un atome d'oxygène.

Les composés de formule X, dans laquelle R3 est un atome d'halogène peuvent être préparés par réaction d'un composé de formule II et d'un halomethyllithien selon la méthode décrite dans Bull. Soc. Chim. Fr.1986, pp 470-477.

Les composés de formule X, dans laquelle R3 est le groupe YR4 peuvent être préparés par réaction d'un composé de formule II et d'un composé HYR4, selon la méthode décrite par Advanced Organic Chemistry Mc Graw Hill Inc. J. March.

Les composés de formule X, dans laquelle R3 est le groupe YR4, dans lequel Y est un atome de soufre, peuvent être préparés par réaction d'un composé de formule II et d'un alkyl(ou aryl) thiomethyllithien selon la méthode décrite dans J. Org. Chem., 1985, vol 95, pp 3429-3431.

Les composés de formule X, dans laquelle R3 est le groupe hydroxyle, peuvent être préparés par réaction d'un composé de formule II avec le paraformaldéhyde selon la méthode décrite dans Tetrahedron Letters, 1992, vol 48, pp 2069-2080.

Les composés de formule XI peuvent être préparés par réaction selon une réaction de cyclopropanation du type Simmons Smith (cf Organic Reactions, 1973, vol 20, p 1-131) avec un dihalogénométhane, de préférence diiodo ou dibromo. Une variante consiste à réaliser la réaction sous ultra-sons comme décrit dans Tetrahedron Letters, 1982, vol 23, pp 2729-2732 ou selon la demande britannique 2 191 774. Dans le cas où R3 est un groupe SR4, dans lequel R4 est un radical alkyle linéaire ou ramifié de 1 à 4 atomes de carbone ou cycloalkyle de 3 à 6 atomes de carbone, on obtient directement à partir des composés de formule X, des composés de formule XII, dans laquelle R'3 représente un halogénure (de préférence un iodure) de méthylsulfonium.

Les composés de formule XII, dans laquelle R'3 est un groupe alkylsulfonate, phénylsulfonate ou naphthylsulfonate, ou halogénoalkylcarbonyloxy peuvent être obtenus à partir d'un composé de formule XI, dans laquelle R3 est le groupe hydroxyle, selon la méthode décrite par Advanced Organic Chemistry Mc Graw Hill Inc. J. March.

Les composés de formule XI peuvent être obtenus directement à partir d'un composé de formule X, dans laquelle R3 est le groupe hydroxyle, ou à partir d'un composé de formule XI, dans laquelle R3 est un groupe alkoxy ou aryloxy selon les méthodes connues, en particulier par action de l'iodure de trimethylsilyle comme décrit dans Silicon Reagents for Organic Synthesis, W.P. Weber, 1983, Springer Verlag.

Les composés de formule XIII peuvent être obtenus des composés de formule XII, par action d'une base telle qu'un hydroxyde alcalin, l'hydrure de sodium ou un alcoolate alcalin dans un solvant tel que le dimethylsulfoxyde, le N,N-dimethylformamide, la N- methyl-2-pyrrolidone, l'acetonotrile, à une température de -10°C à + 60°C.

## SCHEMA REACTIONNEL B(en configuration relative)

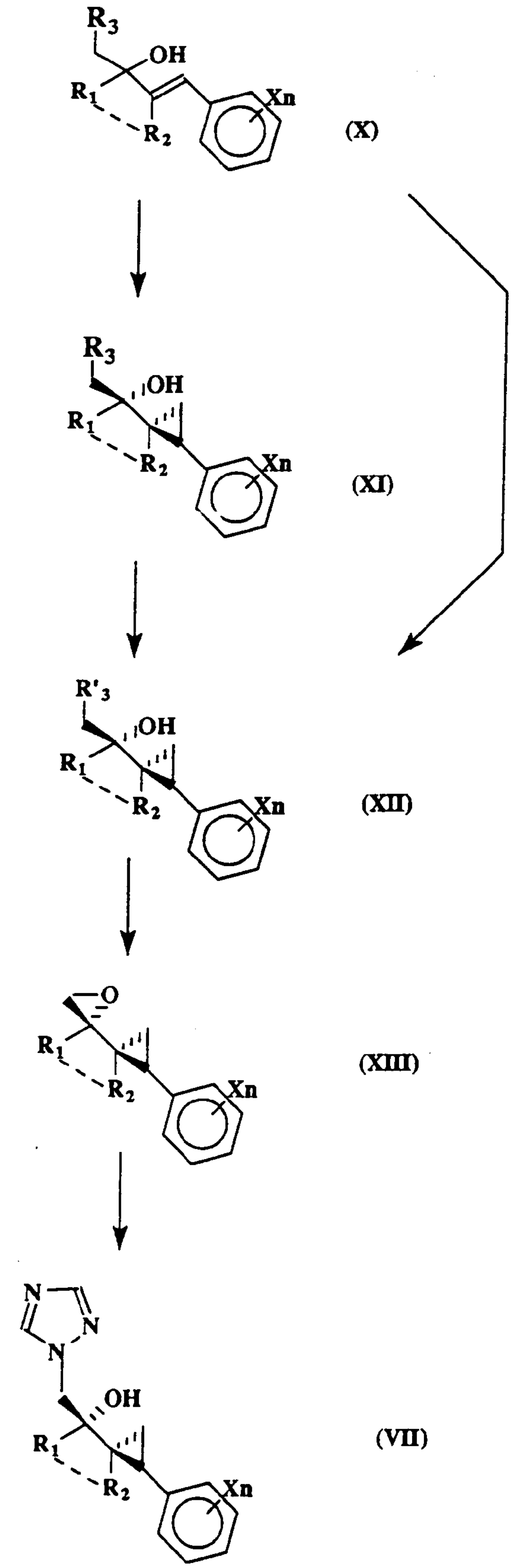

(X)

(XI)

(XII)

(XIII)

(VII)

Les composés de formule VII, dans laquelle E est le groupe méthylene, peuvent être obtenus à partir des composés de formule XIII selon la même méthode que celle décrite précédemment à partir des composés de formule VI.

Les exemples ci-apis sont donnés à titre d'illustration des composés selon l'invention, de leurs procédés de préparation et de leurs propriété antifongiques.

Exemple 1:Préparation du 1-(4-chlorophenyl)-2,4-dimethyl-3-one-pent-1-ène

Du chlorure d'hydrogène est mis à barbotter dans un mélange de 100ml de 2-méthyl-3-pentanone, 113g de 4-chlorobenzaldéhyde et 400ml d'éthanol jusqu'à saturation. La température de la solution est maintenue à 60°C pendant 8h. Le mélange est ensuite versé dans 1l d'eau et extrait avec 500ml d'éther diisopropylique. La phase organique est lavée avec 250ml de soude à 10%, puis avec 250ml de solution saturée de chlorure de sodium et séchée sur sulfate de sodium. Après évaporation, 167g de liquide brun sont obtenus.

Rdt = 93%.

Exemple 2 : Préparation du 1-(4-chlorophenyl)-2-methyl-2-(2-methyl-1-one-1-propyl)-cyclopropane

A une suspension de 61,3g d'hydrure de sodium dans 200ml de NMP chauffée à 50°C, sont ajoutés 185ml d'alcool tert-amylique de manière à avoir un dégagement d'hydrogène régulier. La solution obtenue est refroidie à 25°C et 176g d'iodure de trimethylsulfoxonium solide sont ajoutés, suivis d'une solution de 162g de 1-(4-chlorophenyl)-2,4-dimethyl-3-one-pent-1-ène dans 250ml de NMP. Le mélange résultant est chauffé à 60°C pendant 30mn. Le produit obtenu est laissé dans le milieu réactionnel pour l'étape suivante.

Exemple 3 : Préparation du 1-(4-chlorophenyl)-2-methyl-2-(2-methyl-1-oxirane-1-propyl)-cyclopropane

A la solution précédemment obtenue sont ajoutés 151g de méthylsulfate de trimethylsulfonium dans 200ml de NMP à 25°C. Après 30mn, la réaction est finie. Le composé obtenu est laissé dans le milieu réactionnel pour l'étape suivante.

Exemple 4 : Préparation du 1-(4-chlorophenyl)-2-methyl-2-(2-methyl-1-hydroxy-1-(1,2,4-triazol-1-ylmethyl)-1-propyl)-cyclopropane (composés n° 3 et 4).

A la solution précédemment obtenue, est ajoutée une solution de triazolyl sodium obtenue en mélangeant 60,5g de 1,2,4-triazole et 70g de soude à 50% dans 500ml de NMP. Le mélange réactionnel est chauffé à 130°C pendant 8h. Le mélange est ensuite versé dans 2l d'eau et extrait avec 2 X 750ml d'éther. La phase organique est lavée avec 2l d'eau puis avec 500ml de solution aqueuse saturée de chlorure de sodium et séchée sur sulfate de sodium. Le brut est purifié par chromatographie sur silice avec heptane 60-acétate d'éthyle 37-éthanol 5. 14,5g de diastéréoisomère A (F = 121°-composé 4) et 16,3g de diastéréoisomère B (F = 141°-composé 3) sont obtenus.

Exemple 5: Préparation du 2-(4-chlorophenyl)-4-(1,2,4-triazol-1-ylmethyl)-4-ol-(2,5)-oxaspirooctane (composé n°7).

Un mélange de 9g de 2-(4-chlorobenzylidène)-1-(1,2,4-triazol-1-ylmethyl)-cyclopentan-1-ol et 14,8g d'acide m-chloroperbenzoïque dans 50ml de dichloromethane est agité pendant 3h à température ambiante. 200ml d'eau sont ensuite ajoutés et extraits avec 3 X 100ml de dichloromethane. La phase organique est lavée avec une solution de carbonate de potassium. La phase organique est séchée sur sulfate de sodium et évaporée. Le résidu est cristallisé dans de l'éther diisopropylique pour obtenir 6g de solide blanc (F = 150,5°C)

Rdt = 63%

| • Composé n° | R1 | R2 | W | E | X | F (°C) |
|---|---|---|---|---|---|---|
| 1- (dia B) | iPr | Me | N | $CH_2$ | 2,4-diCl | 147 |
| 2- (dia A) | iPr | Me | N | $CH_2$ | 2,4-diCl | 145 |
| 3- (dia B) | iPr | Me | N | $CH_2$ | 4-Cl | 141 |
| 4- (dia A) | iPr | Me | N | $CH_2$ | 4-Cl | 121 |
| 5- (dia B) | iPr | Me | N | O | 4-Cl | 152 |
| 6- (dia A) | iPr | Me | N | O | 4-Cl | 175 |
| 7 | $-(CH2)_4-$ | | N | O | 4-Cl | 150 |
| 8 | $-(CMe_2CH_2-CH_2)-$ | | N | O | 4-Cl | 143 |
| 9- (dia B) | tBu | Et | N | $CH_2$ | 4-Cl | 117 |
| 10- (dia A) | tBu | Et | N | $CH_2$ | 4-Cl | 100 |
| 11- (dia B) | iPr | Et | N | $CH_2$ | 4-Cl | 131 |
| 12- (dia A) | iPr | Et | N | $CH_2$ | 4-Cl | 109 |
| 13- (2 dia) | $-(CMe_2CH_2-CH_2)-$ | | N | $CH_2$ | 4-Cl | 113 |
| 14-(dia A) | $-(CHMeCH_2-CH_2-CH_2)-$ | | N | $CH_2$ | 4-Cl | 183 |
| 15- (dia A) | $-(CH2)_4-$ | | N | $CH_2$ | 4-Cl | 165 |
| 16- (dia B) | $-(CH2)_4-$ | | N | $CH_2$ | 4-Cl | 150 |
| 17- (dia A) | $-(CH2)_5-$ | | N | $CH_2$ | 4-Cl | 119 |
| 18- (dia B) | $-(CH2)_5-$ | | N | $CH_2$ | 4-Cl | 124 |
| 19- (dia A) | $-(CHMeCH_2-CH_2)-$ | | N | $CH_2$ | 4-Cl | 128 |
| 20- (dia B) | $-(CHMeCH_2-CH_2)-$ | | N | $CH_2$ | 4-Cl | 155 |
| 21- (diaC) | $-(CHMeCH_2-CH_2)-$ | | N | $CH_2$ | 4-Cl | 157 |
| 22- (diaB) | $-(CHMeCH_2-CH_2)-$ | | N | $CH_2$ | 4-Cl | 114 |
| 23- (diaC) | $-(CHMeCH_2-CH_2)-$ | | N | $CH_2$ | 4-Cl | 161 |
| 24- (diaD) | $-(CHMeCH_2-CH_2)-$ | | N | $CH_2$ | 4-Cl | 179 |
| 25 | $-(CH_2)_3-$ | | N | $CH_2$ | 4-Cl | 123 |

Exemple 6: Préparation du 2-(-4-chlorobenzylidene)-5-methyl-1-(mehtylthiomethyl)cyclopentanol:

Dans un ballon de 500ml équipé d'une ampoule de coulée isobare de 100ml et d'une agitation magnétique, on introduit sous argon 69ml (0,011 mole) de n-butyllithium 1,6M. On refroidit le balon dans un bain de glace avant de couler successivement 16,6 ml(0,11 mole) de tetramethylethylene diamine puis, après 15 mn, 8,1 ml (0,11 mole) de dimethylsulfure.

Après 3 heures d'agitation à température ambiante, le ballon est refroidi à -70°C, avant de couler 20g (0,09 mole) de 2-(4-chlorobenzylidene)-5-methylcyclopentanone dissoute dans 100 ml de tetrahydrofurane sec.

Après une demi-heure, à -70,°C, on introduit dans le ballon 5g de chlorure d'ammonium puis 80 ml d'éther éthylique. La solution est alors hydrolysée avec 200 ml d'eau saturée en chlorure d'ammonium, extraite avec 2 fois 60 ml d'éther. Les phases organiques sont lavées, avec 3 fois 80 ml d'eau saturée en chlorure de sodium puis séchées sur sulfate de magnésium.

On recueille, après élimination des solvants, 25 g( rendement brut :98%) de 2-(4chlorobenzylidene) 5-methyl-1-(methylthiomethyl) cyclopentanol, sous la forme d'une huile visqueuse jaune clair présentant 2 diastéréoisomères dans les proportions 70/30.

Exemple 7: Préparation de l'iodure de (1-(4-chlorophenyl)-5-methyl spiro(2,4) heptan-4-ol)-4-yl dimethyl sulfonium):

Dans un ballon de 250 ml équipé d'un réfrigérant, d'une ampoule de coulée isobare et d'une agitation mécanique, on introduit sous azote 11g (0,168 at.g) de zinc en poudre dans 60 ml de glyme sec. Le ballon est alors introduit dans une cuve sous ultrasons(modèle Branson 2200) pendant 1h 30mn avant d'introduire rapidement 13,5g(0,048 mole) du composé préparé à l'exemple 6, dilué dans 10 ml de glyme. Ensuite, sous ultrasons, on coule goutte à goutte à 45°C, 13,7 ml(0,170 mole) de diiodomethane dilué avec 10ml de glyme. On note une exothermie importante de 10 à 15°C pendant l'introduction du diiodométhane. Le mélange est maintenu 5 h, sous ultrasons, à 55°C.

Après avoir laissé la température revenir à l'ambiante, on ajoute 100ml d'acétate d'ethyle avant d'hydrolyser avec 150 ml d'une solution d'eau saturée en chlorure d'ammonium. La phase aqueuse est extraite avec 3 fois 70 ml d'acétate d'ethyle. Les phases organiques rassemblées sont lavées, avec 3 fois 80 ml d'eau saturée en chlorure de sodium, séchées sur sulfate de magnésium et concentrées.

On recueille 35g d'une huile visqueuse jaune clair, qui est triturée avec un mélange 50/50 d'éther éthylique/éther diisopropylique et conduit à un précipité beige très hygroscopique. Après séchage à 40°C on obtient 18g (rendement: 87%)d'une masse sous forme d'une meringue blanche fondant à 78°C.

Exemple 8: Préparation du (1-(4-chlorophenyl)-4-(1H-1,2,4-triazolyl-1-ylmethyl)-5-methyl spiro(2,4) heptan-4-ol)(composé 19 et 21):

Dans un ballon de 500 ml équipé d'un réfrigérant, d'une ampoule de coulée de 100 ml et d'une agitation mécanique, on introduit160 ml de dimethylformamide sec puis 1,64 g(0,04 mole) d'hydrure de sodium à 60% dans l'huile. On ajoute 7,64 g(0,084 mole) de triazolyl-sodium préparé et dilué dans 40 ml de dimethylformamide.

Le milieu est alors chauffé à 85°C pendant 3 heures. Apis avoir laissé la température revenir à l'ambiante, le milieu est hydrolysé et traité comme à l'exemple 4.

On obtient ainsi, après élimination des solvants, 10,5g d'un résidu visqueux brun, qui, par chromatographie sur colonne de silice(éluant:chlorure de methylene/methanol 98/2), conduit à 5,9g d'un mélange des composés 19 et 21 dans les proportions 30/70(rendement global: 41 %)

Le composé 21 est obtenu par recristallisation du mélange précédent avec le binaire acétate d'éthyle/éther diisopropylique. Point de fusion: 157°C.

Les composés 2, 4, 10, 12, 16, 17, 22, 24, et 25 peuvent être également obtenus selon ce procédé.

Exemple 9: Test in vivo sur Botrytis cinerea(pourriture grise) sur feuille excisée de tomate(souches sensibles et résistantes aux benzimidazoles

On prépare, par broyage fin, une suspension aqueuse de la matière active à tester ayant la composition suivante :

- matière active: 60 mg
- agent tensioactif Tween 80, (oléate de dérivé polyoxyéthyléné du sorbitan) dilué à 10% dans l'eau : 0,3 ml

- on complète à 60 ml d'eau pour obtenir une suspension/solution à 1g/l.

Cette suspension aqueuse est ensuite éventuellement diluée par de l'eau pour obtenir la concentration désirée en matière active.

Des tomates culitvées en serre(variété Marmande agées de 30 jours sont traitées par pulvérisation avec des suspensions aqueuses telles que définies ci-dessus et à diverses concentrations du composé à tester.

Des plants, utilisés comme témoins sont traités par une solution aqueuse ne contenant pas la matière active.

Au bout de 24 heures les feuilles sont coupées et mises dans une boîte de Pétri (diamètre 14 cm), dont le fond a été préalablement garni d'un disque de papier filtre humide (10 folioles par boîte).

L'inoculum est ensuite apporté à l'aide d'une seringue par dépôt de gouttes (3 par foliole) d'une suspension de spores de Botrytis cinerea, sensibles aux benzimidazoles ou résistantes aux benzimidazoles, obtenue à partir de cultures de 15 jours, mises ensuite en suspension à raison de 150 000 unités par $cm^{3}$.

Le contrôle est fait 6 jours après la contamination en comparaison avec un témoin non traité.

Dans ces conditions, on observe, à la dose de 1 g/l, une protection bonne ( au moins 75%) ou totale avec les composés 1, 3, 4, 5, 6, 7, 8, 10, 12, 13, 16, 17, 19, 22 et 23 contre les souches de Botrytis sensibles aux benzimidazoles.

Exemple 10 : Test in vivo sur *Pyricularia oryzae* responsable de la piriculariose du riz :

On prépare, par broyage fin, une suspension aqueuse de la matière active à tester ayant la composition suivante :

- matière active: 60 mg
- agent tensioactif Tween 80, (oléate de dérivé polyoxyéthyléné du sorbitan) dilué à 10% dans l'eau : 0,3 ml
- on complète à 60 ml d'eau pour obtenir une suspension/solution à 1 g/l.

Cette suspension aqueuse est ensuite éventuellement diluée par de l'eau pour obtenir la concentration désirée en matière active.

Du riz, semé en godets dans un mélange 50/50 de tourbe enrichie et de pouzzolane, est traité au stade 10 cm de hauteur par pulvérisation de la suspension aqueuse ci dessus.

Des plants, utilisés comme témoins sont traités par une solution aqueuse ne contenant pas la matière active.

Au bout de 24 heures, on applique sur les feuilles une suspension aqueuse de spores de Pyricularia oryzae, obtenues à partir d'une culture de 15 jours, mises ensuite en suspension à raison de 100 000 unités par $cm^3$.

Les plants de riz sont placés pendant 24 heures en incubation (25°C, 100% d'humidité relative), puis mis en cellule d'observation, dans les mêmes conditions, pendant 5 jours.

La lecture se fait 6 jours après la contamination.

Dans ces conditions, on observe, à la dose de 1 g/l, une protection bonne (au moins 75%) ou totale avec les composés 2, 3, 4, 5, 6, 8, 10, 11, 12, 13, 14, 16, 17, 19, 21, 22, 23 et 24.

Exemple 11 : Test in vivo sur *Erysiphe graminis* sur orge (oïdium de l'orge) :

On prépare, par broyage fin, une suspension aqueuse de la matière active à tester ayant la composition suivante:

- matière active: 60 mg
- agent tensioactif Tween 80, (oléate de dérivé polyoxyéthyléné du sorbitan) dilué à 10% dans l'eau: 0,3 ml
- on complète à 60 ml d'eau pour obtenir une suspension/solution à 1 g/l.

Cette suspension aqueuse est ensuite éventuellement diluée par de l'eau pour obtenir la concentration désirée en matière active.

De l'orge, en godets, semée sur un substrat tourbe terre pouzzolane 50/50, est traitée au stade 10 cm de hauteur par pulvérisation de la suspension aqueuse ci dessus.

Des plants, utilisés comme témoins sont traités par une solution aqueuse ne contenant pas la matière active.

Au bout de 24 heures, on saupoudre les plants d'orge avec des spores d'Erysiphe graminis, le saupoudrage étant effectué à l'aide de plants malades.

La lecture se fait 7 à 14 jours apis la contamination, en comparaison avec les plants témoins.

Dans ces conditions, on observe, à la dose de 1 g/l, une protection bonne (au moins 75%) ou totale avec

les composés 1, 2, 4, 5, 6, 8, 10, 12, 13, 16, 19, 20, 21, 22, 23 et 24.

Exemple 12 : Test in vivo sur *Puccinia recondita* (rouille du blé) :

On prépare, par broyage fin, une suspension aqueuse de la matière active à tester ayant la composition suivante:

- matière active: 60 mg
- agent tensioactif Tween 80, (oléate de dérivé polyoxyéthyléné du sorbitan) dilué à 10% dans l'eau: 0,3 ml
- on complète à 60 ml d'eau pour obtenir une suspension/solution à 1 g/l.

Cette suspension aqueuse est ensuite éventuellement diluée par de l'eau pour obtenir la concentration désirée en matière active.

Du blé, en godets, semé sur un substrat tourbe terre pouzzolane 50/50, est traité au stade 10 cm de hauteur par pulvérisation de la suspension aqueuse ci dessus.

Des plants, utilisés comme témoins sont traités par une solution aqueuse ne contenant pas la matière active.

Au bout de 24 heures, une suspension aqueuse de spores (100000 sp/$cm^3$) est pulvérisée sur le blé; cette suspension a été obtenue à partir de plants contaminés. On place ensuite le blé pendant 24 heures en cellule d'incubation à environ 20°C et à 100% d'humidité relative, puis pendant 7 à 14 jours à 60% d'humidité relative.

Le contrôle de l'état des plants se fait entre le 8ème et le 15ème jour après la contamination, par comparaison avec un témoin non traité.

Dans ces conditions, on observe, à la dose de 1 g/l, une protection bonne (au moins 75%) ou totale avec les composés suivants 1, 3, 4, 6, 7, 8, 9, 10, 11, 12 13, 16, 22, 23, et 24.

Ces résultats montrent clairement les bonnes propriétés fongicides des dérivés selon l'invention contre les maladies des plantes dues à des champignons appartenant aux familles les plus diverses telles que les Phycomycètes, les Basidiomycètes, les ascomycètes, les adelomycètes ou fungi imperfecti, en particulier les Botrytis sp., Piricularia oryzae, les oïdium(Erysiphe sp.) les rouilles( Puccinia sp.), le mildiou de la vigne.

Pour leur emploi pratique, les composés selon l'invention sont rarement utilisés seuls. Le plus souvent ces composés font partie de compositions. Ces compositions, utilisables comme agents fongicides, contiennent comme matière active un composé selon l'invention tel que décrit précédemment en mélange avec les supports solides ou liquides, acceptables en agriculture et les agents tensio-actifs également acceptables en agriculture. En particulier sont utilisables les supports inertes et usuels et les agents tensio-actifs usuels. Ces compositions font également partie de l'invention.

Ces compositions peuvent contenir aussi toute sorte d'autres ingrédients tels que, par exemple, des colloïdes protecteurs, des adhésifs, des épaississants, des agents thixotropes, des agents de pénétration, des stabilisants, des séquestrants, etc... Plus généralement les composés utilisés dans l'invention peuvent être combinés à tous les additifs solides ou liquides correspondant aux techniques habituelles de la mise en formulation.

D'une façon générale, les compositions selon l'invention contiennent habituellement de 0,05 à 95 % environ (en poids) d'un composé selon l'invention (appelé par la suite matière active), un ou plusieurs supports solides ou liquides et, éventuellement, un ou plusieurs agents tensioactifs.

Par le terme "support", dans le présent exposé, on désigne une matière organique ou minérale, naturelle ou synthétique, avec laquelle le composé est combiné pour faciliter son application sur la plante, sur des graines ou sur le sol. Ce support est donc généralement inerte et il doit être acceptable en agriculture, notamment sur la plante traitée. Le support peut être solide (argiles, silicates naturels ou synthétiques, silice, résines, cires, engrais solides, etc...) ou liquide (eau, alcools, notamment le butanol etc...).

L'agent tensioactif peut être un agent émulsionnant, dispersant ou mouillant de type ionique ou non ionique ou un mélange de tels agents tensioactifs. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés, des esters d'acides gras et de polyols, les dérivés à fonction sulfates, sulfonates et phosphates des composés précédents. La présence d'au moins un agent tensioactif est généralement indispensable lorsque le composé et/ou le support inerte ne sont pas solubles dans l'eau et que l'agent vecteur de l'application est l'eau.

Ainsi donc, les compositions à usage agricole selon l'invention peuvent contenir les matières actives selon l'invention dans de très larges limites, allant de 0,05 % à 95 % (en poids). Leur teneur en agent tensio-actif

est avantageusement comprise entre 5 % et 40 % en poids.

Ces compositions selon l'invention sont elles-mêmes sous des formes assez diverses, solides ou liquides.

Comme formes de compositions solides, on peut citer les poudres pour poudrage (à teneur en composé pouvant aller jusqu'à 100 %) et les granulés, notamment ceux obtenus par extrusion, par compactage, par imprégnation d'un support granulé, par granulation à partir d'une poudre (la teneur en composé dans ces granulés étant entre 0,5 et 80 % pour ces derniers cas), les composés ou tablettes effervescents.

Les composés de formule (I) peuvent encore être utilisés sous forme de poudres pour poudrage ; on peut aussi utiliser une composition comprenant 50 g de matière active et 950 g de talc ; on peut aussi utiliser une composition comprenant 20 g de matière active, 10 g de silice finement divisée et 970 g de talc ; on mélange et broie ces constituants et on applique le mélange par poudrage.

Comme formes de compositions liquides ou destinées à constituer des compositions liquides lors de l'application, on peut citer les solutions, en particulier les concentrés solubles dans l'eau, les concentrés émulsionnables, les émulsions, les suspensions concentrées, les aérosols, les poudres mouillables (ou poudre à pulvériser), les pâtes, les gels.

Les concentrés émulsionnables ou solubles comprennent le plus souvent 10 à 80 % de matière active, les émulsions ou solutions prêtes à l'application contenant, quant à elles, 0,001 à 20 % de matière active.

En plus du solvant, les concentrés émulsionnables peuvent contenir quand c'est nécessaire, 2 à 20 % d'additifs appropriés comme les stabilisants, les agents tensio-actifs, les agents de pénétration, les inhibiteurs de corrosion, les colorants ou les adhésifs précédemment cités.

A partir de ces concentrés, on peut obtenir par dilution avec de l'eau des émulsions de toute concentration désirée, qui conviennent particulièrement à l'application sur les cultures.

A titre d'exemple, voici la composition de quelques concentrés émulsionnables :

| Exemple CE 1 : | |
|---|---|
| - matière active | 400 g/l |
| - dodécylbenzène sulfonate alcalin | 24 g/l |
| - nonylphénol oxyéthylé à 10 molécules d'oxyde d'éthylène | 16 g/l |
| - cyclohexanone | 200 g/l |
| - solvant aromatique | q.s.p.1 litre |

Selon une autre formule de concentré émulsionnable, on utilise :

| Exemple CE 2 | |
|---|---|
| - matière active | 250 g |
| - huile végétale époxydée | 25 g |
| - mélange de sulfonate d'alcoylaryle et d'éther de polyglycol et d'alcools gras | 100 g |
| - diméthylformamide | 50 g |
| - xylène | 575 g |

Les suspensions concentrées, également applicables en pulvérisation, sont préparées de manière à obtenir un produit fluide stable ne se déposant pas et elles contiennent habituellement de 10 à 75 % de matière active, de 0,5 à 15 % d'agents tensioactifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide organique dans lequel la matière active est peu ou pas soluble : certaines matières solides organiques ou des sels minéraux peuvent être dissous dans le support pour aider à empêcher la sédimentation ou comme antigels pour l'eau.

A titre d'exemple, voici une composition de suspension concentrée :

| Exemple SC 1 : | |
|---|---|
| - matière active | 500 g |
| - phosphate de tristyrylphénol polyéthoxylé | 50 g |
| - alkylphénol polyéthoxylé | 50 g |
| - polycarboxylate de sodium | 20 g |
| - éthylène glycol | 50 g |
| - huile organopolysiloxanique (antimousse) | 1 g |
| - polysaccharide | 1,5 g |
| - eau | 316,5 g |

Les poudres mouillables (ou poudre à pulvériser) sont habituellement préparées de manière qu'elles contiennent 20 à 95 % de matière active, et elles contiennent habituellement, en plus du support solide, de 0 à 30 % d'un agent mouillant, de 3 à 20 % d'un agent dispersant, et, quand c'est nécessaire, de 0,1 à 10 % d'un ou plusieurs stabilisants et/ou autres additifs, comme des agents de pénétration, des adhésifs, ou des agents antimottants, colorants, etc...

Pour obtenir les poudres à pulvériser ou poudres mouillables, on mélange intimement les matières actives dans les mélangeurs appropriés avec les substances additionnelles et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et ces suspensions sont utilisables trés avantageusement en particulier pour l'application sur les feuilles des végétaux.

A la place des poudres mouillables, on peut réaliser des pâtes. Les conditions et modalités de réalisation et d'utilisation de ces pâtes sont semblables à celles des poudres mouillables ou poudres à pulvériser.

A titre d'exemple, voici diverses compositions de poudres mouillables (ou poudres à pulvériser) :

| Exemple PM 1 | |
|---|---|
| - matière active | 50% |
| - alcool gras éthoxylé (agent mouillant) | 2,5% |
| - phényléthylphénol éthoxylé (agent dispersant) | 5% |
| - craie (support inerte) | 42,5% |

| Exemple PM 2 : | |
|---|---|
| - matière active | 10% |
| - alcool synthétique oxo de type ramifié, en C13 éthoxylé par 8 à 10 oxyde d'éthylène (agent mouillant) | 0,75% |
| - lignosulfonate de calcium neutre (agent dispersant) | 12% |
| -carbonate de calcium (charge inerte) | q.s.p. 100 % |

<u>Exemple PM 3</u> :

Cette poudre mouillable contient les mêmes ingrédients que dans l'exemple précédent, dans les proportions ci-après :

| | |
|---|---|
| - matière active | 75% |
| - agent mouillant | 1,50% |
| - agent dispersant | 8% |
| - carbonate de calcium (charge inerte) | q.s.p. 100% |

| Exemple PM 4 : | |
|---|---|
| - matière active | 90% |
| - alcool gras éthoxylé (agent mouillant) | 4% |
| - phényléthylphénol éthoxylé (agent dispersant) | 6% |

| Exemple PM 5 : | |
|---|---|
| - matière active | 50% |
| - mélange de tensio-actifs anioniques et non ioniques (agent mouillant) | 2,5% |
| - lignosulfonate de sodium (agent dispersant) | 5% |
| - argile kaolinique (support inerte) | 42,5% |

Les dispersions et émulsions aqueuses, par exemple les compositions obtenues en diluant à l'aide d'eau une poudre mouillable ou un concentré émulsionnable selon l'invention, sont comprises dans le cadre général de la présente invention. Les émulsions peuvent être du type eau-dans-l'huile ou huile-dans-l'eau et elles peuvent avoir une consistance épaisse comme celle d'une "mayonnaise".

Les composés selon l'invention peuvent être formulés sous la forme de granulés dispersibles dans l'eau également compris dans le cadre de l'invention.

Ces granulés dispersibles, de densité apparente généralement comprise entre environ 0,3 et 0,6 ont une dimension de particules généralement comprise entre environ 150 et 2000 et de préférence entre 300 et 1500 microns.

La teneur en matière active de ces granulés est généralement comprise entre environ 1 % et 90 %, et de préférence entre 25 % et 90 %.

Le reste du granulé est essentiellement composé d'une charge solide et éventuellement d'adjuvants tensio-actifs conférant au granulé des propriétés de dispersibilité dans l'eau. Ces granulés peuvent être essentiellement de deux types distincts selon que la charge retenue est soluble ou non dans l'eau. Lorsque la charge est hydrosoluble, elle peut être minérale ou, de préférence, organique. On a obtenu d'excellents résultats avec l'urée. Dans le cas d'une charge insoluble, celle-ci est de préférence minérale, comme par exemple le kaolin ou la bentonite. Elle est alors avantageusement accompagnée d'agents tensio-actifs (à raison de 2 à 20 % en poids du granulé) dont plus de la moitié est, par exemple, constituée par au moins un agent dispersant, essentiellement anionique, tel qu'un polynaphtalène sulfonate alcalin ou alcalino terreux ou un lignosulfonate alcalin ou alcalino-terreux, le reste étant constitué par des mouillants non ioniques ou anioniques tel qu'un alcoyl naphtalène sulfonate alcalin ou alcalino-terreux.

Par ailleurs, bien que cela ne soit pas indispensable, on peut ajouter d'autres adjuvants tels que des agents anti-mousse.

Le granulé selon l'invention peut être préparé par mélange des ingrédients nécessaires puis granulation selon plusieurs techniques en soi connues (drageoir, lit fluide, atomiseur, extrusion, etc...). On termine généralement par un concassage suivi d'un tamisage à la dimension de particule choisie dans les limites mentionnés ci-dessus. On peut encore utilisé des granulés obtenus comme précédemment puis imprégnés avec une composition contenant la matière active.

De préférence, il est obtenu par extrusion, en opérant comme indiqué dans les exemples ci-après.

Exemple GD1 : Granulés dispersibles

Dans un mélangeur, on mélange 90 % en poids de matière active et 10 % d'urée en perles. Le mélange est ensuite broyé dans un broyeur à broches. On obtient une poudre que l'on humidifie avec environ 8 % en poids d'eau. La poudre humide est extrudée dans une extrudeuse à rouleau perforé. On obtient un granulé qui est séché, puis concassé et tamisé, de façon à ne garder respectivement que les granulés d'une dimension comprise entre 150 et 2000 microns.

Exemple GD2 : Granulés dispersibles

Dans un mélangeur, on mélange les constituants suivants :

| | |
|---|---|
| - matière active | 75% |
| - agent mouillant (alkylnaphtalène sulfonate de sodium) | 2% |
| - agent dispersant (polynaphtalène sulfonate de sodium) | 8% |
| - charge inerte insoluble dans l'eau (kaolin) | 15% |

Ce mélange est granulé en lit fluide, en présence d'eau, puis séché, concassé et tamisé de manière à obtenir des granulés de dimension comprise entre 0,15 et 0,80 mm.

Ces granulés peuvent être utilisés seuls, en solution ou dispersion dans de l'eau de manière à obtenir la dose cherchée. Ils peuvent aussi être utilisés pour préparer des associations avec d'autres matières actives, notamment fongicides, ces dernières étant sous la forme de poudres mouillables, ou de granulés ou suspensions aqueuses.

En ce qui concerne les compositions adaptées au stockage et au transport, elles contiennent plus avantageusement de 0,5 à 95 % (en poids) de substance active.

L'invention a également pour objet l'utilisation des composés selon l'invention pour la lutte contre les maladies fongiques des plantes par traitement préventif ou curatif de ces dernières ou de leur lieu de **croissance**.

## Revendications

**1)** Composés à groupement triazole ou imidazole, caractérisés en ce qu'ils répondent à la formule générale VII :

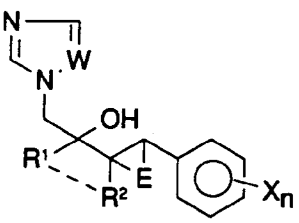

dans laquelle :

- $R^1$ représente un radical alkyle de 1 à 4 atomes de carbone, linéaire ou ramifié; cycloalkyle de 3 à 6 atomes de carbone; akylidène, hydroxyalkyle, carboxyalkyle chacun contenant de 1 à 4 atomes de carbone et étant éventuellement substitué par un ou plusieurs atomes d'halogène; aryle, choisi dans le groupe comprenant phényl, naphtyl, éventuellement substitué par un ou plusieurs groupes choisis parmi halogène, nitro, cyano, amino, alkyle ou alkoxy de 1 à 4 atomes de carbone éventuellement substitués par un ou plusieurs atomes d'halogène, alkylamino dont la partie alkyle contient de 1 à 4 atomes de carbone éventuellement substitués par un ou plusieurs atomes d'halogène; aralkyle, dont la partie alkyle possède de 1 à 4 atomes de carbone, linéaire ou ramifié et dont la partie aryle est définie comme ci-dessus;
- $R^2$ représente un radical alkyle ou perhalogénoalkyle de 1 à 4 atomes de carbone, linéaire ou ramifié ou un atome d'halogène, de préférence chlore ou fluor, lorsque E est différent d'un atome d'oxygène;
- $R^1$ et $R^2$, ensemble, peuvent former un radical $-(CH_2)_3-$, $-(CH_2)_4-$, $-(CH_2)_5-$, conduisant ainsi à un cycle à 5, 6 ou 7 atomes de carbone, cycle dont chaque hydrogène peut éventuellement être substitué par un

radical choisi dans le groupe comprenant les atomes d'halogène, les radicaux alkyle, de 1 à 4 atomes de carbone, linéaire ou ramifié, éventuellement substitués par un ou plusieurs atomes d'halogène;

- W est un radical CH ou l'atome d'azote;
- E représente un atome d'oxygène ou un radical $CH_2$;
- X représente un atome d'halogène; un radical alkyle, de 1 à 4 atomes de carbone, linéaire ou ramifié, éventuellement substitués par un ou plusieurs atomes d'halogène; alkylamino, la partie alkyl ayant de 1 à 4 atomes de carbone éventuellement substitués par un ou plusieurs atomes d'halogène; alcoxy de 1 à 4 atomes de carbone éventuellement substitués par un ou plusieurs atomes d'halogène; phénoxy ou benzyloxy éventuellement substitué par un ou plusieurs halogènes; nitro; cyano;
- n est un entier égal à 1, 2, 3, 4 ou 5, avec, quand cet entier est supérieur ou égal à 2, la possibilité que les groupements X ne soient pas identiques;

et les formes salifiées de ces composés, acceptables en agriculture.

**2)** Composés selon la revendication 1, caractérisés en ce que W est l'atome d'azote.

**3)** Composés selon la revendication 2, caractérisés en ce que le cycle incluant E est un oxirane.

**4)** Composés selon la revendication 2, caractérisés en ce que le cycle incluant E est un cyclopropane.

**5)** Composés selon l'une des revendications 3 ou 4, caractérisés en ce que X est un atome d'halogène.

**6)** Composés selon la revendication 5, caractérisés en ce que X est l'atome de chlore.

**7)** Composés selon la revendication 2, caractérisés en ce que R1 est un radical propyl ou butyl, linéaire ou ramifié, et R2 est un radical méthyl ou éthyl.

**8)** Composés selon la revendication 2, caractérisés en ce que R1 et R2 formentnesemble un cycle de 5 ou 6 atomes de carbone.

**9)** Compositions fongicides, caractérisés en ce qu'elles comprennent, en association avec un ou des supports solides ou liquides acceptables en agriculture et/ou des agents tensio-actifs également acceptables en agriculture, comme matière active, au moins un composé de fomule générale VII :

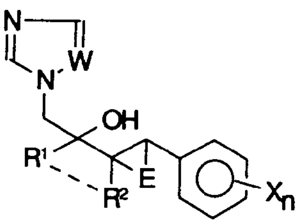

dans laquelle :

- $R^1$ représente un radical alkyle de 1 à 4 atomes de carbone, linéaire ou ramifié; cycloalkyle de 3 à 6 atomes de carbone; alkylidène, hydroxyalkyle, carboxyalkyle de 1 à 4 atomes de carbone, ces radicaux étant éventuellement substitués par un ou plusieurs atomes d'halogène; aryle, choisi dans le groupe comprenant phényl, naphtyl, éventuellement substitué par un ou plusieurs groupes choisis parmi halogène, nitro, cyano, amino, alkyle ou alkoxy de 1 à 4 atomes de carbone éventuellement substitués par un ou plusieurs atomes d'halogène, alkylamino, la partie alkyl ayant de 1 à 4 atomes de carbone éventuellement substitués par un ou plusieurs atomes d'halogène; aralkyle, dont la partie alkyle possède de 1 à 4 atomes de carbone, linéaire ou ramifié et dont la partie aryle est définie comme ci-dessus;
- $R^2$ représente un radical alkyle ou perhalogénoalkyle de 1 à 4 atomes de carbone, linéaire ou ramifié ou un atome d'halogène, de préférence chlore ou fluor, lorsque E est différent d'un atome d'oxygène;
- $R^1$ et $R^2$, ensemble, peuvent former un radical $-(CH_2)_3-$, $-(CH_2)_4-$, $-(CH_2)_5-$, conduisant ainsi à un cycle à 5, 6 ou 7 atomes de carbone, cycle dont chaque hydrogène peut éventuellement être substitué par un radical choisi dans le groupe comprenant les atomes d'halogène, les radicaux alkyle, de 1 à 4 atomes de carbone, linéaire ou ramifié, éventuellement substitués par un ou plusieurs atomes d'halogène;
- W est un radical CH ou l'atome d'azote;
- E représente un atome d'oxygène ou un radical $CH_2$;
- X représente un atome d'halogène, un radical alkyle, de 1 à 4 atomes de carbone, linéaire ou ramifié, éventuellement substitué par un ou plusieurs atomes d'halogène; alkylamino, la partie alkyl ayant de 1 à 4 atomes de carbone éventuellement substitués par un ou plusieurs atomes d'halogène; alcoxy de 1 à 4 atomes de carbone éventuellement substitués par un ou plusieurs atomes d'halogène; phénoxy ou benzyloxy éventuellement substitué par un ou plusieurs halogènes; nitro; cyano;
- n est un entier égal à 1, 2, 3, 4 ou 5, avec, quand cet entier est supérieur ou égal à 2, la possibilité que les groupements X ne soient pas identiques;

et les formes salifiées de ces composés.

**10)** Compositions fongicides selon la revendication 9, caractérisées en ce que la matière active est un composé selon l'une des revendications 2 à 7.

**11)** Compositions fongicides selon l'une des revendications 9 ou 10, caractérisées en ce que le ou les composés selon l'une des revendications 1 à 7 est présent à raison de 0,5 à 95% en poids.

**12)** Procédé de traitement des cultures atteintes ou susceptibles d'être atteintes par les maladies fongiques caractérisé en ce l'on applique, de façon préventive ou curative, une quantité efficace d'un composé selon l'une des revendications 1 à 7 ou d'une composition selon l'une des revendications 8 à 10.

**13)** Procédé de traitement des cultures selon la revendication 12, caractérisé en ce que la dose efficace est comprise entre 0,002 et 5 kg/ha.

**14)** Procédé de traitement des cultures selon la revendication 13, caractérisé en ce que la dose efficace est comprise entre 0,005 et 1 kg/ha.

**15)** Produit de multiplication des plantes cultivées, carctérisé en ce qu'il a subi un traitement de protection par un, ou plusieurs, des composés selon l'une des revendications 1 à 8 ou par une composition selon l'une des revendications 9 à 11.

**16)** Produit de multiplication des plantes selon la revendication 15, caractérisé en ce qu'il est constitué d'un graine ayant subi un traitement de protection à raison de 0,1 à 500 g par quintal.

**17)** Composés utiles notamment comme intermédiaires dans la préparation des composés selon les revendications 1 à 8, caractérisés en ce qu'ils répondent aux formules V, VI, et VIII :

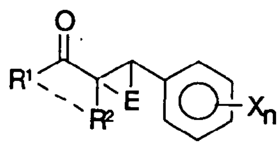
(V)

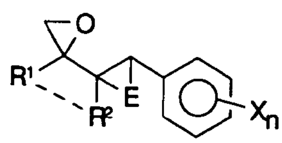
(VI)

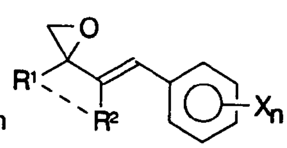
(VIII)

dans lesquelles W, E, R1, R2, X et n ont la même signification que dans la formule générale de la revendication 1.

**18)** Procédé de préparation des composés de formule VII selon l'une des revendications 1 à 8 caractérisé en ce que l'on fait réagir un oxirane de formule VI avec un hétérocycle azoté en présence de base et dans un solvant à température comprise entre +25°C et le reflux du solvant :

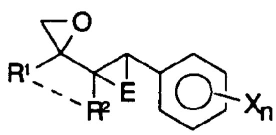
(VI)

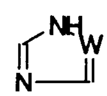

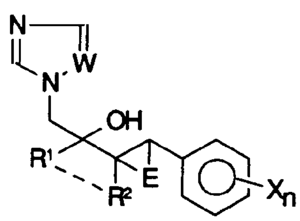
(VII)

les substituants ayant la même signification que dans la revendication 1

**19)** Procédé de préparation des composés oxiranes de formule VI, caractérisé en ce que un composé de formule V est mis à réagir avec un ylure de sulfonium de formule $(CH_3)_2S=CH_2$ dans un solvant à une température comprise entre -10 et +50°C :

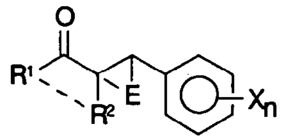

(V)

les substituants ayant la même signification que dans la revendication 1.

**20)** Procédé de préparation des composés de formule V, caractérisé en ce que l'on fait réagir un ylure de sulfoxonium du composé de formule $(CH_3)_2S(O)=CH_2$ sur un composé de formule II dans un solvant à une température comprise entre 20 et 100°C :

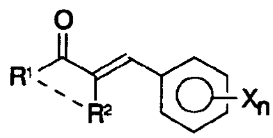

(II)

les substituants ayant la même signification que dans la revendication 1.

**21)** Procédé de préparation des composés de formule V, caractérisé en ce que l'on fait réagir un peroxyde ou hydroperoxyde en présence de base dans un mélange eau-alcool sur un composé de formule II:

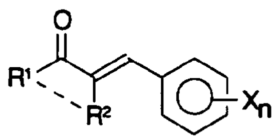

(II)

les substituants ayant la même signification que dans la revendication 1, à une température comprise entre 0°C et le reflux du solvant.

**22)** Procédé de préparation des composés de formule VII selon l'une des revendications 1 à 8, caractérisé en ce que l'on fait réagir de l'acide métachloro perbenzoïque ou de l'hydroperoxyde de ter-butyle sur un composé de formule IX dans un solvant tel que les solvants halogénés ou les hydrocarbures:

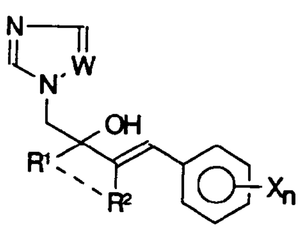

(IX)

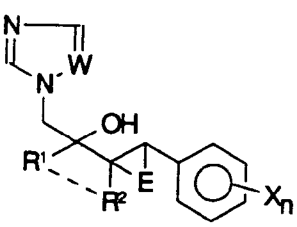

(VII)

les substituants ayant la même signification que dans la revendication 1

**23)** Procédé de préparation des composés de formule IX par action d'un hétérocycle azoté, en présence de base et dans un solvant à température comprise entre +25°C et le reflux du solvant, sur un oxirane de formule VIII :

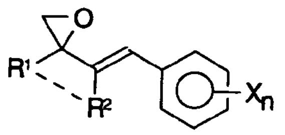

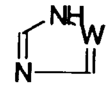

(VIII)

les substituants ayant la même signification que dans la revendication 1

**24)** Procédé de préparation des composés de formule VIII, carctérisé en ce qu'on fait réagir un ylure de sulfonium du composé de formule $(CH_3)_2S{=}CH_2$ dans un solvant à une température comprise entre -10 et +50°C sur un composé de formule II :

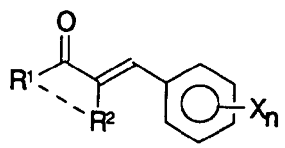

(II)

les substituants ayant la même signification que dans la revendication 1.

**25)** Procédé de préparation des composés de formule VII selon l'une des revendications 1 à 7, caractérisé en ce que l'on fait réagir un oxiranede formule XIII avec un hétérocycle azoté en présence de base et dans un solvant à température comprise entre +25°C et le reflux du solvant:

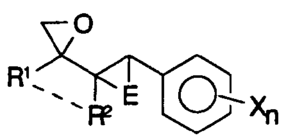

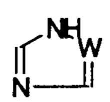

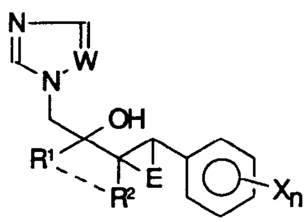

(XIII) (VII)

<u>à remplacer par les formules stéréo</u>

les substituants ayant la même signification que dans la revendication 1.

**26)** Procédé de préparation des composés de formule VII, selon l'une des revendications 1 à 8, dans laquelle E est le groupe méthylene, caractérisé en ce que l'on fait réagir des composés oxirane de formule XIII avec un hétérocycle azoté en présence de base et dans un solvant à température comprise entre +25°C et le reflux du solvant.

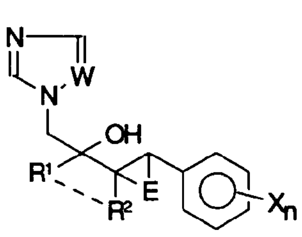

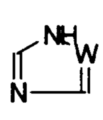

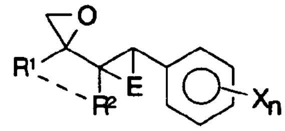

(XIII) (VII)

introduire les formules stéréo

**27)** Composés utiles notamment comme intermédiaires dans la préparation des composés selon les revendications 1 à 7 caractérisés en ce qu'ils répondent aux formules X, XI, XII et XIII:

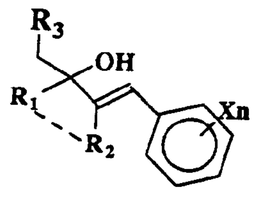
(X)

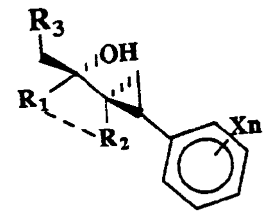
(XI)

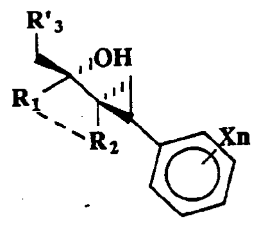
(XII)

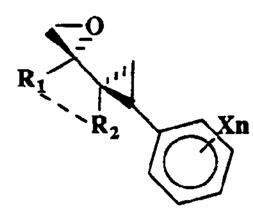
(XIII)

dans lesquelles:

- W, E, R1, R2, X et n ont la même signification que dans la formule générale de la'revendication 1;
- R3 représente un atome d'halogène, de préférence de chlore ou de brome, ou un groupe Y-R4, dans lequel Y représente un atome de d'oxygène, de soufre ou d'azote et R4 représente un atome d'hydrogène, un radical alkyle linéaire ou ramifié de 1 à 4 atomes de carbone, cycloalkyle de 3 à 6 atomes de carbone, phényl ou napthyl, éventuellement substitué par 1 à 5 substituants choisi dans le groupe comprenant un atome d'halogène, amino, alkyle ou alkoxy de 1 à 4 atomes de carbone, ou encore phénylalkyle ou naphthylalkyle, dont la partie alkyle comprend de 1 à 4 atomes de carbone. Dans le cas où Y est un atome d'azote, il porte en plus de R4 un second substituant, identique ou différent de R4 et pouvant avoir les mêmes significations et
- R'3 représente un atome d'halogène(identique à R3, quand R3 est un atome d'halogène), ou
  - un sel de sulfonium ou de sulfoxonium, de préférence C1-C4 alkylsulfonium ou C1-C4 alkyl sulfoxonium, lorsque Y est un atome de soufre,
  - un sel d'ammonium(de préférence C1-C4 alkylammonium ), lorsque Y est un atome d'azote ou
  - un groupe alkyl(de préférence méthyl)sulfonate, phényl(de préférence tolyl)sulfonate ou naphthylsulfonate, halogénoalkylcarbonyloxy(de préférence trifluoromethylcarbonyloxy), lorsque Y est un atome d'oxygène.

**28)** Procédé de traitement des cultures atteintes ou susceptibles d'être atteintes par les maladies fongiques caractérisé en ce l'on applique sur les feuilles une dose efficace d'un composé selon l'une des revendications 1 à 8.

Office européen des brevets

# RAPPORT DE RECHERCHE EUROPEENNE

Numero de la demande

EP 93 42 0148

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X,P | EP-A-0 483 616 (BASF A.G.)<br>* table 2, pages 9- 13 * | 1 | C07D521/00<br>C07D405/08 |
| X,P | * table 1, pages 6-7 * | 27 | C07D405/06<br>C07C49/237 |
| X,P | EP-A-0 486 409 (RHONE-POULENC AGROCHIMIE)<br>* page 12; exemple Vb * | 27 | C07D303/08<br>C07D303/12<br>C07D407/04 |
| X | EP-A-0 433 780 (BASF A.G.)<br>* table 2, pages 19-23 * | 17 | |
| Y | * table 4, pages 32 et suiv. * | 1-28 | |
| X,D | EP-A-0 378 953 (RHONE-POULENC AGROCHIMIE)<br>* composé (IX) et exemples * | 27 | |
| Y,D | * le document en entier * | 1-28 | |
| X | EP-A-0 333 059 (BASF A.G.)<br>* composé (II) et exemples * | 17 | |
| Y | * le document en entier * | 1-28 | |
| Y | EP-A-0 390 022 (BASF A.G.)<br>* composé (II) et exemples pages 5-6 * | 1-28 | |
| D | & DE-A-3 909 862 | | |
| Y | EP-A-0 345 639 (BASF A.G.)<br>* composé (II) et exemples pages 5-6 * | 1-28 | |
| D | & DE-A-3 819 053 | | |
| Y,D | EP-A-0 212 605 (HOECHST A.G.)<br>* composé (IV), page 4 et exemples pages 21-26 * | 1-28 | |
| A | EP-A-0 341 954 (KUREHA KAGAKU KOGYO KABUSHIKI KAISHA)<br>* le document en entier * | 1-28 | |
| A | WO-A-8 905 581 (E.I. DU PONT DE NEMOURS)<br>* pages 106-122, table 4 * | 1-28 | |

DOMAINES TECHNIQUES RECHERCHES (Int. Cl.5)

C07D
C07C
A01N
A61N

Le présent rapport a été établi pour toutes les revendications

| Lieu de la recherche | Date d'achèvement de la recherche | Examinateur |
|---|---|---|
| BERLIN | 07 JUILLET 1993 | FRELON D. L. M. F. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)